(19) Europäisches Patentamt / European Patent Office / Office européen des brevets

(11) **EP 4 378 918 A1**

(12) **EUROPEAN PATENT APPLICATION**
published in accordance with Art. 153(4) EPC

(43) Date of publication:
**05.06.2024 Bulletin 2024/23**

(21) Application number: **22849525.5**

(22) Date of filing: **27.07.2022**

(51) International Patent Classification (IPC):
**C07C 5/25** (2006.01)   **C07C 11/02** (2006.01)
**C07C 303/06** (2006.01)   **C07C 303/32** (2006.01)
**C07C 309/20** (2006.01)   **C07B 61/00** (2006.01)
**B01J 21/04** (2006.01)

(52) Cooperative Patent Classification (CPC):
**B01J 21/04; C07B 61/00; C07C 5/25; C07C 11/02;
C07C 303/06; C07C 303/32; C07C 309/20**

(86) International application number:
**PCT/JP2022/028887**

(87) International publication number:
**WO 2023/008464 (02.02.2023 Gazette 2023/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.07.2021   JP 2021122422**

(71) Applicant: **Kao Corporation
Chuo-ku
Tokyo 103-8210 (JP)**

(72) Inventors:
• **KITASHIMA,Kota
Wakayama-shi, Wakayama 640-8580 (JP)**
• **YAMADA,Yudai
Wakayama-shi, Wakayama 640-8580 (JP)**
• **NAKAMURA,Noriyoshi
Wakayama-shi, Wakayama 640-8580 (JP)**

(74) Representative: **Hoffmann Eitle
Patent- und Rechtsanwälte PartmbB
Arabellastraße 30
81925 München (DE)**

(54) **METHOD FOR PRODUCING INTERNAL OLEFIN, METHOD FOR PRODUCING INTERNAL OLEFIN SULFONATE, AND LOW-TEMPERATURE STABILIZATION METHOD**

(57)   The present invention provides a method for producing an internal olefin for obtaining an internal olefin sulfonate having high low-temperature stability, a method for producing an internal olefin sulfonate using the internal olefin, and a low-temperature stabilization method. The method for producing an internal olefin of the present invention includes the step of internally isomerizing an $\alpha$-olefin having 8 or more and 24 or less carbon atoms using a fixed bed reactor in which solid acid catalyst particles are packed in a reaction tube. A ratio (D/dp) of an inner diameter D of the reaction tube to an average particle diameter dp of the solid acid catalyst particles is 25 or more.

EP 4 378 918 A1

**Description**

TECHNICAL FIELD

The present invention relates to a method for producing an internal olefin, a method for producing an internal olefin sulfonate using the internal olefin, and a low-temperature stabilization method.

BACKGROUND ART

Conventionally, anionic surfactants, particularly alkyl sulfates and polyoxyalkylene alkyl ether sulfates, have been widely used as household and industrial washing components because of their excellent detergency and foaming power. As one of the anionic surfactants, a report has been made about an olefin sulfonate, particularly an internal olefin sulfonate obtained by using an internal olefin having a double bond not at any terminal of the olefin but at an inside thereof as a raw material.

The internal olefin sulfonate is generally obtained by sulfonating an internal olefin by reacting the internal olefin with sulfur trioxide, neutralizing the obtained sulfonated product, and further preferably hydrolyzing the neutralized product.

For example, Patent Document 1 proposes a method for producing an internal olefin sulfonate. The method includes the steps of: obtaining an internal olefin from a primary aliphatic alcohol having 8 or more and 24 or less carbon atoms; sulfonating the internal olefin to obtain a sulfonated product; and neutralizing the sulfonated product and then hydrolyzing the neutralized product.

PRIOR ART DOCUMENT

PATENT DOCUMENT

Patent Document 1: JP-A-2014-156462

SUMMARY OF THE INVENTION

PROBLEMS TO BE SOLVED BY THE INVENTION

However, the conventional internal olefin sulfonate has low low-temperature stability, and has a problem that a precipitate is generated when the internal olefin sulfonate is stored in a low-temperature environment of around 0°C.

The present invention provides a method for producing an internal olefin for obtaining an internal olefin sulfonate having high low-temperature stability, a method for producing an internal olefin sulfonate using the internal olefin, and a low-temperature stabilization method.

MEANS FOR SOLVING THE PROBLEMS

The present invention relates to a method for producing an internal olefin,
the method comprising the step of internally isomerizing an α-olefin having 8 or more and 24 or less carbon atoms using a fixed bed reactor in which solid acid catalyst particles are packed in a reaction tube, wherein a ratio (D/dp) of an inner diameter D of the reaction tube to an average particle diameter dp of the solid acid catalyst particles is 25 or more.

The present invention also relates to a method for producing an internal olefin sulfonate,
the method comprising the step of sulfonating the internal olefin obtained by the method for producing an internal olefin.

The present invention further relates to a method for stabilizing an internal olefin sulfonate at a low temperature,
the method comprising the steps of: sulfonating the internal olefin obtained by the method for producing an internal olefin; and neutralizing the sulfonated internal olefin to obtain the internal olefin sulfonate.

EFFECT OF THE INVENTION

According to the present invention, in the step of internally isomerizing an α-olefin having 8 or more and 24 or less carbon atoms, the ratio (D/dp) of the inner diameter D of a reaction tube of a fixed bed reactor to the average particle diameter dp of solid acid catalyst particles is adjusted to 25 or more, and therefore an internal olefin for obtaining an internal olefin sulfonate having high low-temperature stability can be produced. The internal olefin sulfonate produced using the internal olefin obtained by the production method of the present invention has high low-temperature stability, and is characterized in that a precipitate is hardly generated even when stored in a low-temperature environment of around 0°C. The reason is not clear, but it is considered that the double bond position of the internal olefin as a raw material and the content ratio of internal olefins having different double bond positions affect the low-temperature stability of the internal olefin sulfonate.

MODE FOR CARRYING OUT THE INVENTION

Hereinafter, the present invention will be described in detail.

<Method for producing internal olefin>

A method for producing an internal olefin of the present invention includes the step of internally isomerizing an $\alpha$-olefin having 8 or more and 24 or less carbon atoms using a fixed bed reactor in which solid acid catalyst particles are packed in a reaction tube.

The internal olefin is an olefin having a double bond other than the terminal of a carbon chain, that is, an olefin having a double bond at a position of 2 or more of the carbon chain. The internal isomerization of the $\alpha$-olefin means that the $\alpha$-olefin is converted to an olefin (internal olefin) having a double bond at a position other than the terminal of the carbon chain by converting a double bond at an $\alpha$-position to the position of 2 or more of the carbon chain.

The number of carbon atoms in the $\alpha$-olefin is preferably 10 or more, more preferably 12 or more, and still more preferably 14 or more, and preferably 22 or less, and more preferably 18 or less, from the viewpoint of washing performance and the like when an internal olefin sulfonate is used as a detergent.

In the step of internally isomerizing the $\alpha$-olefin, a fixed bed reactor in which solid acid catalyst particles are packed in a reaction tube can be used.

From the viewpoint of increasing the conversion rate of the $\alpha$-olefin to the internal olefin, the solid acid catalyst particles are preferably solid acid catalyst particles containing one or more elements selected from aluminum, iron, and gallium, more preferably solid acid catalyst particles containing aluminum, and still more preferably $\gamma$-alumina and/or aluminum phosphate.

The average particle diameter dp (sphere equivalent diameter) of the solid acid catalyst particles is preferably 0.5 mm or more, and more preferably 1 mm or more, and preferably 6 mm or less, and more preferably 5 mm or less, from the viewpoint of handleability, catalytic activity, and the relationship with the inner diameter D of the reaction tube.

The packing height of the solid acid catalyst particles is preferably 100 mm or more, and more preferably 1000 mm or more, from the viewpoint of the production efficiency of the internal olefin. The upper limit value of the packing height is preferably 8000 mm or less from the viewpoint of the installation space of the reaction tube.

The inner diameter D of the reaction tube may be, for example, 10 mm or more. The inner diameter D of the reaction tube is preferably 50 mm or more, and more preferably 500 mm or more, and preferably 2500 mm or less, and more preferably 2000 mm or less, from the viewpoint of the production efficiency of the internal olefin, and the relationship with the average particle diameter dp of the solid acid catalyst particles.

The fixed bed reactor is preferably a fixed bed reactor in which a reaction tube is installed in a vertical direction, from the viewpoint of the handleability of the solid acid catalyst particles and the installation space of the fixed bed reactor. The fixed bed reactor is preferably a mono-tubular fixed bed reactor having one reaction tube from the viewpoint of simplifying equipment and reducing a pressure drop, and is preferably a multi-tubular fixed bed reactor having a plurality of reaction tubes from the viewpoint of removing heat of reaction.

In the method for producing an internal olefin of the present invention, the average particle diameter dp of the solid acid catalyst particles and the inner diameter D of the reaction tube are adjusted such that the ratio (D/dp) of the inner diameter D of the reaction tube to the average particle diameter dp of the solid acid catalyst particles is 25 or more. D/dp is preferably 200 or more, and more preferably 400 or more, from the viewpoint of obtaining an internal olefin which is a raw material for producing an internal olefin sulfonate having high low-temperature stability. The upper limit value of D/dp is preferably 2000 or less, more preferably 1500 or less, and still more preferably 1000 or less, from the viewpoint of the handleability of the solid acid catalyst particles and the installation space of the fixed bed reactor.

A reaction temperature when the $\alpha$-olefin is internally isomerized is preferably 300°C or lower, more preferably 290°C or lower, and still more preferably 280°C or lower, from the viewpoint of obtaining an internal olefin which is a raw material for producing an internal olefin sulfonate having high low-temperature stability. The lower limit value of the reaction temperature is preferably 100°C or higher, more preferably 150°C or higher, and still more preferably 170°C or higher, from the viewpoint of increasing the conversion rate of the $\alpha$-olefin to the internal olefin.

A pressure when the $\alpha$-olefin is internally isomerized is preferably 0.03 MPa or more, more preferably 0.05 MPa or more, and still more preferably 0.1 MPa or more, and preferably 1 MPa or less, more preferably 0.5 MPa or less, still more preferably 0.2 MPa or less, in terms of an absolute pressure, from the viewpoint of increasing the conversion rate of the $\alpha$-olefin to the internal olefin and from the viewpoint of obtaining the internal olefin which is a raw material for producing the internal olefin sulfonate having high low-temperature stability.

In the step of internally isomerizing the α-olefin, an inert gas may be introduced into the fixed bed reactor. Examples of the inert gas include nitrogen, argon, and helium, and nitrogen is preferable from the viewpoint of easy availability. From the viewpoint of improving a reaction rate and productivity, the introduction amount of the inert gas is preferably 0.1 mol or more, and more preferably 0.2 mol or more, and preferably 10 mol or less, and more preferably 1 mol or less, based on 1 mol of the α-olefin as a raw material.

In the step of internally isomerizing the α-olefin, WHSV (α-olefin supply mass per hour per solid acid catalyst unit mass) is preferably 0.03/hr or more, more preferably 0.05/hr or more, still more preferably 0.07/hr or more, and yet still more preferably 0.09/hr or more, and is preferably 7/hr or less, more preferably 5/hr or less, still more preferably 2.5/hr or less, yet still more preferably 2/hr or less, further preferably 1.5/hr or less, and further more preferably 1/hr or less, from the viewpoint of reaction efficiency and the viewpoint of obtaining an internal olefin which is a raw material for producing an internal olefin sulfonate having high low-temperature stability.

In the step of internally isomerizing the α-olefin, LHSV (liquid hourly space velocity) is preferably 0.03/hr or more, more preferably 0.05/hr or more, and still more preferably 0.07/hr or more, and is preferably 5/hr or less, more preferably 4/hr or less, still more preferably 2.5/hr or less, yet still more preferably 2/hr or less, further preferably 1.5/hr or less, and further more preferably 1/hr or less, from the viewpoint of reaction efficiency and the viewpoint of obtaining an internal olefin which is a raw material for producing an internal olefin sulfonate having high low-temperature stability.

The internal olefin obtained in the above step may be purified by distillation or the like in order to remove an α-olefin as a raw material and by-products such as a dimer.

The average double bond position of the internal olefin is preferably 2 or more, more preferably 3 or more, still more preferably 3.5 or more, and yet still more preferably 4 or more, and preferably 4.8 or less, more preferably 4.6 or less, and still more preferably 4.5 or less, from the viewpoint of obtaining an internal olefin sulfonate having high low-temperature stability and from the viewpoint of washing performance and foaming performance when the internal olefin sulfonate is used as a detergent.

The internal olefins may be used alone or in combination of two or more. When two or more kinds of internal olefins are used in combination, the internal olefin having 16 carbon atoms and the internal olefin having 18 carbon atoms are preferably used in combination from the viewpoint of washing performance and the like when the internal olefin sulfonate is used as a detergent.

<Method for producing internal olefin sulfonate>

A method for producing an internal olefin sulfonate of the present invention includes the step of sulfonating the internal olefin obtained by the production method. The sulfonation step is preferably a sulfonation step of reacting the internal olefin obtained by the production method with sulfur trioxide to obtain a sulfonated product. The method for producing an internal olefin sulfonate of the present invention preferably includes a neutralization step of neutralizing the obtained sulfonated product to obtain a neutralized product, and further preferably includes the step of hydrolyzing the obtained neutralized product to obtain a hydrolyzed product.

In the present invention, the internal olefin sulfonate is a sulfonate obtained by sulfonating the internal olefin, preferably neutralizing the sulfonated internal olefin, and more preferably hydrolyzing the neutralized internal olefin. That is, when the internal olefin is sulfonated, the internal olefin is converted into β-sultone, γ-sultone, and olefin sulfonic acid, and these are further converted into hydroxysulfonate and olefin sulfonate through a neutralization step and preferably a hydrolysis step. The obtained product is mainly a mixture of these. In the present invention, these products, and mixtures thereof are collectively referred to as an internal olefin sulfonate.

<Sulfonation step>

The sulfonation step is the step of sulfonating the internal olefin, and is preferably the step of reacting the internal olefin with sulfur trioxide to obtain a sulfonated product.

Sulfur trioxide is preferably caused to react as sulfur trioxide gas from the viewpoint of improving the reactivity thereof. The sulfur trioxide gas is preferably diluted to 1 to 30 vol% with air and an inert gas such as nitrogen, and more preferably diluted to 1.5 to 10 vol%.

The amount of sulfur trioxide used is preferably 0.8 mol or more, more preferably 0.9 mol or more, and still more preferably 0.95 mol or more, based on 1 mol of the internal olefin, from the viewpoint of improving the yield of the sulfonated product, and is preferably 1.2 mol or less, more preferably 1.1 mol or less, and still more preferably 1.05 mol or less, from the viewpoint of the economic efficiency thereof and from the viewpoint of suppressing the coloring of the sulfonated product.

In the sulfonation reaction for causing the internal olefin to react with sulfur trioxide, it is preferable to use a thin-film type sulfonation reactor provided with an external jacket from the viewpoint of causing the internal olefin in a liquid form to react with sulfur trioxide in a gaseous form.

A treatment temperature in the sulfonation step is preferably 0°C or higher from the viewpoint of preventing the coagulation of sulfur trioxide and the sulfonated product, and is preferably 50°C or lower from the viewpoint of suppressing the coloring of the sulfonated product.

Since the sulfonation reaction is an exothermic reaction, it is preferable to provide an external jacket in the sulfonation reactor and pass cooling water thereinto to cool the sulfonation reactor. The temperature of the cooling water, which is passed into the external jacket of the sulfonation reactor is preferably 0 °C or higher from the viewpoint of improving the reaction rate. The temperature is preferably 30°C or lower, and more preferably 20°C or lower, from the viewpoint of suppressing any side reaction to reduce impurities such as the internal olefin and any inorganic salt in the finally obtained internal olefin sulfonate.

The sulfonation reaction ratio is preferably 95% or more, more preferably 97% or more, and still more preferably 98% or more, from the viewpoint of improving the yield of the sulfonated product, and is preferably 99.8% or less from the viewpoint of suppressing the coloring of the sulfonated product caused by excessive sulfur trioxide.

<Neutralization step>

The neutralization step is the step of neutralizing the sulfonated product obtained in the sulfonation step to obtain a neutralized product.

The alkali compound used for neutralization may be an inorganic alkali compound or an organic alkali compound. As the inorganic alkali compound, for example, alkali metal hydroxides such as sodium hydroxide and potassium hydroxide, and alkali metal carbonates such as sodium carbonate and potassium carbonate can be used. As the organic alkali compound, for example, ammonia, and amine compounds having 1 to 6 carbon atoms such as 2-aminoethanol can be used.

The alkali compound used for neutralization is preferably an inorganic alkali compound, and more preferably an alkali metal hydroxide, from the viewpoint of the availability thereof and economic efficiency. Among them, at least one selected from sodium hydroxide and potassium hydroxide is still more preferable, sodium hydroxide is yet still more preferable from the viewpoint of the ease of use, and potassium hydroxide is further preferable from the viewpoint of the low-temperature stability.

The alkali compound used for neutralization is preferably used as an aqueous alkali solution from the viewpoint of the handleability. The concentration of the aqueous alkali solution is preferably 1% by mass or more, more preferably 4.5% by mass or more, still more preferably 7% by mass or more, yet still more preferably 10% by mass or more, and further preferably 12% by mass or more, from the viewpoint of the economic efficiency and the viewpoint of suppressing the production of impurities such as the internal olefin and inorganic salts. The concentration is preferably 30% by mass or less, more preferably 25% by mass or less, still more preferably 23% by mass or less, yet still more preferably 20% by mass or less, and further preferably 15% by mass or less, from the viewpoint of the productivity in the hydrolysis step.

The amount of the alkali compound used for neutralization is preferably 1 time by mole or more, and more preferably 1.03 times by mole or more, relative to the sulfonic acid group from the viewpoint of suppressing the production of impurities such as the internal olefin and inorganic salts and from the viewpoint of improving the reactivity, and is preferably 2.5 times by mole or less, more preferably 2.0 times by mole or less, and still more preferably 1.5 times by mole or less, from the viewpoint of the economic efficiency and from the viewpoint of suppressing the production of impurities such as the internal olefin and inorganic salts.

In the neutralization step, a temperature when the sulfonate is mixed with the aqueous alkali solution and a temperature during the neutralization reaction are preferably 40°C or lower, more preferably 35°C or lower, still more preferably 30°C or lower, and yet still more preferably 25°C or lower, from the viewpoint of suppressing the production of impurities such as the internal olefin and inorganic salts, due to side reactions, and are preferably 0°C or higher, more preferably 10°C or higher, still more preferably 15°C or higher, and yet still more preferably 20°C or higher, from the viewpoint of improving the reactivity.

In the neutralization step, any type of mixer may be used as long as the mixer can efficiently mix the sulfonated product with the aqueous alkali solution and can apply a shear force to the oily product. Examples of the mixer include a static mixer, a collision type mixer, an agitating impeller type mixer, and a vibration type mixer. Examples of the static mixer include a static mixer manufactured by Noritake Co., Ltd. Examples of the collision type mixer include a high-pressure emulsifying machine manufactured by Nanomizer Inc. Examples of the agitating impeller type mixer include a Milder manufactured by Matsubo Corporation, and a Homo Mixer manufactured by PRIMIX Corporation. Among them, the agitating impeller type mixer is preferable from the viewpoint of machine costs.

From the viewpoint of the productivity and suppression of the production of by-products, the neutralization step is preferably performed by a so-called continuous method in which the sulfonated product and the aqueous alkali solution are added while the reaction liquid is circulated using a loop type reactor, and at the same time, the reaction liquid is extracted.

In the neutralization step, a neutralization time is preferably 5 minutes or more, more preferably 8 minutes or more, still more preferably 10 minutes or more, and yet still more preferably 12 minutes or more, from the viewpoint of sufficiently performing the neutralization reaction. From the viewpoint of improving the productivity, the time is preferably 100 minutes or less, more preferably 50 minutes or less, still more preferably 40 minutes or less, yet still more preferably 30 minutes or less, further preferably 20 minutes or less, and further more preferably 15 minutes or less.

In the case of the continuous method, the neutralization time can be represented as an average residence time obtained by dividing the volume of the loop type reactor by the total addition amount of the sulfonated product and the aqueous alkali solution per unit time. The average residence time is preferably 8 minutes or more, more preferably 10 minutes or more, and still more preferably 12 minutes or more, from the viewpoint of suppressing the heat of neutralization, and is preferably 100 minutes or less, more preferably 60 minutes or less, still more preferably 40 minutes or less, yet still more preferably 30 minutes or less, further preferably 20 minutes or less, and further more preferably 15 minutes or less, from the viewpoint of improving the productivity. In the case of the continuous method, the circulation ratio of the reaction liquid is preferably 3 times or more, more preferably 6 times or more, and still more preferably 9 times or more, from the viewpoint of improving the reactivity, and is preferably 30 times or less, more preferably 20 times or less, and still more preferably 15 times or less, from the viewpoint of suppressing an increase in the pressure in the reactor. Here, the circulation ratio is a ratio of the amount of the entire contents circulating in the reactor to the flowing amount of the reaction liquid charged into the reactor, and is represented by (the total circulation amount in the reactor)/(the amount charged into the reactor).

In the neutralization step, a water-soluble organic solvent such as alcohol or acetone, and a surfactant such as polyoxyethylene alkyl ether or α-olefin sulfonic acid can be caused to coexist. It is preferable not to use the coexisting product since the product is denatured during the neutralizing reaction, remains in the final product, or gives a burden to the purification step. In the case of using the product, the product is used in an amount of preferably 20% by mass or less, more preferably 10% by mass or less, still more preferably 2% by mass or less, and yet still more preferably 1% by mass or less in the mixed liquid.

<Hydrolysis step>

The production method of the present invention preferably includes a hydrolysis step of hydrolyzing the obtained neutralized product.

In the hydrolysis step, a temperature during the hydrolysis is preferably 120°C or higher, more preferably 140°C or higher, and still more preferably 160°C or higher, from the viewpoint of improving the reactivity, and is preferably 220°C or lower, and more preferably 180°C or lower, from the viewpoint of suppressing the decomposition of the product.

The hydrolysis reaction may be conducted in a batch reactor or in a continuous reactor.

The treatment time of the hydrolysis step is preferably 30 minutes or more, and more preferably 45 minutes or more, from the viewpoint of completing the reaction, and is preferably 4 hours or less, more preferably 3.5 hours or less, still more preferably 3 hours or less, yet still more preferably 2 hours or less, and further preferably 90 minutes or less, from the viewpoint of improving the productivity.

The concentration of the aqueous internal olefin sulfonate solution obtained in the hydrolysis step is preferably 15% by mass or more, more preferably 30% by mass or more, still more preferably 35% by mass or more, yet still more preferably 40% by mass or more, further preferably 45% by mass or more, further more preferably 48% by mass or more, and further still more preferably 50% by mass or more, from the viewpoint of the productivity, and is preferably 75% by mass or less, more preferably 70% by mass or less, still more preferably 65% by mass or less, and yet still more preferably 60% by mass or less, from the viewpoint of the viscosity and the like of the aqueous solution.

The internal olefin sulfonate can be used as it is for various applications, but the sulfonate may be further subjected to purification such as desalting or decoloring.

The internal olefin sulfonate obtained by the production method of the present invention has high low-temperature stability, and hardly generates a precipitate even when stored in a low-temperature environment of around 0 °C. Since the internal olefin sulfonate has good purity and hue, the internal olefin sulfonate can be suitably used for various applications such as body detergents, shampoos, clothing detergents, and tableware detergents.

<Low-temperature stabilization method>

The internal olefin obtained by the production method of the present invention is sulfonated and neutralized to obtain an internal olefin sulfonate, and therefore the obtained internal olefin sulfonate can be stabilized at a low temperature. The internal olefin and the internal olefin sulfonate refer to the internal olefin and internal olefin sulfonate described above. The low-temperature stabilization means that a state change hardly occurs at room temperature or lower, and can be confirmed by, for example, a decrease in the production of precipitates at a temperature of -5°C under normal pressure.

The present invention and preferred embodiments of the present invention are described below.

<1> A method for producing an internal olefin,
the method comprising the step of internally isomerizing an α-olefin having 8 or more and 24 or less carbon atoms using a fixed bed reactor in which solid acid catalyst particles are packed in a reaction tube, wherein a ratio (D/dp) of an inner diameter D of the reaction tube to an average particle diameter dp of the solid acid catalyst particles is 25 or more.

<2> The method for producing an internal olefin according to <1>, wherein the α-olefin preferably has a carbon number of 10 or more, more preferably 12 or more, and still more preferably 14 or more, and preferably 22 or less, and more preferably 18 or less.

<3> The method for producing an internal olefin according to <1> or <2>, wherein the solid acid catalyst particles are preferably solid acid catalyst particles containing one or more elements selected from aluminum, iron, and gallium, more preferably solid acid catalyst particles containing aluminum, and still more preferably γ-alumina and/or aluminum phosphate.

<4> The method for producing an internal olefin according to any one of <1> to <3>, wherein the average particle diameter dp of the solid acid catalyst particles is preferably 0.5 mm or more, and more preferably 1 mm or more, and preferably 6 mm or less, and more preferably 5 mm or less.

<5> The method for producing an internal olefin according to any one of <1> to <4>, wherein a packing height of the solid acid catalyst particles is preferably 100 mm or more, and more preferably 1000 mm or more, and preferably 8000 mm or less.

<6> The method for producing an internal olefin according to any one of <1> to <5>, wherein a packing height is preferably 1000 mm or more and 8000 mm or less.

<7> The method for producing an internal olefin according to any one of <1> to <6>, wherein the inner diameter D of the reaction tube may be 10 mm or more, preferably 50 mm or more, and more preferably 500 mm or more, and preferably 2500 mm or less, and more preferably 2000 mm or less.

<8> The method for producing an internal olefin according to any one of <1> to <7>, wherein the fixed bed reactor is a fixed bed reactor in which a reaction tube is installed in a vertical direction, and preferably a mono-tubular fixed bed reactor or a multi-tubular fixed bed reactor.

<9> The method for producing an internal olefin according to any one of <1> to <8>, wherein the ratio (D/dp) of the inner diameter D of the reaction tube to the average particle diameter dp of the solid acid catalyst particles is preferably 200 or more, and more preferably 400 or more.

<10> The method for producing an internal olefin according to any one of <1> to <9>, wherein the upper limit value of D/dp is preferably 2000 or less, more preferably 1500 or less, and still more preferably 1000 or less.

<11> The method for producing an internal olefin according to any one of <1> to <10>, wherein the D/dp is preferably 200 or more and 2000 or less, more preferably 400 or more and 1500 or less, and still more preferably 400 or more and 1000 or less.

<12> The method for producing an internal olefin according to any one of <1> to <11>, wherein a reaction temperature when the α-olefin is internally isomerized is 300°C or lower.

<13> The method for producing an internal olefin according to any one of <1> to <12>, wherein a reaction temperature when the α-olefin is internally isomerized is preferably 300°C or lower, more preferably 290°C or lower, and still more preferably 280°C or lower.

<14> The method for producing an internal olefin according to any one of <1> to <13>, wherein the lower limit value of the reaction temperature when the α-olefin is internally isomerized is preferably 100°C or higher, more preferably 150°C or higher, and still more preferably 170°C or higher.

<15> The method for producing an internal olefin according to any one of <1> to <14>, wherein a pressure when the α-olefin is internally isomerized is preferably 0.03 MPa or more, more preferably 0.05 MPa or more, and still more preferably 0.1 MPa or more, and preferably 1 MPa or less, more preferably 0.5 MPa or less, still more preferably 0.2 MPa or less, in terms of an absolute pressure.

<16> The method for producing an internal olefin according to any one of <1> to <15>, wherein an inert gas may be introduced into the fixed bed reactor in the step of internally isomerizing the α-olefin, and nitrogen is preferable as the inert gas.

<17> The method for producing an internal olefin according to <16>, wherein the introduction amount of the inert gas is preferably 0.1 mol or more, and more preferably 0.2 mol or more, and preferably 10 mol or less, and more preferably 1 mol or less, based on 1 mol of the α-olefin as a raw material.

<18> The method for producing an internal olefin according to any one of <1> to <17>, wherein in the step of internally isomerizing, WHSV (α-olefin supply mass per hour per solid acid catalyst unit mass) is 0.03/hr or more and 7/hr or less.

<19> The method for producing an internal olefin according to any one of <1> to <18>, wherein in the step of internally isomerizing the α-olefin, WHSV is preferably 0.03/hr or more, more preferably 0.05/hr or more, still more preferably 0.07/hr or more, and yet still more preferably 0.09/hr or more, and is preferably 7/hr or less, more preferably 5/hr or less, still more preferably 2.5/hr or less, yet still more preferably 2/hr or less, further preferably 1.5/hr or less, and further more preferably 1/hr or less.

<20> The method for producing an internal olefin according to any one of <1> to <19>, wherein the WHSV is preferably 0.03/hr or more and 7/hr or less, more preferably 0.05/hr or more and 5/hr or less, still more preferably 0.07/hr or more and 2.5/hr or less, yet still more preferably 0.09/hr or more and 2/hr or less, further preferably 0.09/hr or more and 1.5/hr or less, and further more preferably 0.09/hr or more and 1/hr or less.

<21> The method for producing an internal olefin according to any one of <1> to <20>, wherein in the step of internally isomerizing, LHSV (liquid hourly space velocity) is 0.03/hr or more and 5/hr or less.

<22> The method for producing an internal olefin according to any one of <1> to <21>, wherein in the step of internally isomerizing the $\alpha$-olefin, LHSV (liquid hourly space velocity) is preferably 0.03/hr or more, more preferably 0.05/hr or more, and still more preferably 0.07/hr or more, and is preferably 5/hr or less, more preferably 4/hr or less, still more preferably 2.5/hr or less, yet still more preferably 2/hr or less, further preferably 1.5/hr or less, and further more preferably 1/hr or less.

<23> The method for producing an internal olefin according to any one of <1> to <22>, wherein the LHSV is preferably 0.03/hr or more and 5/hr or less, more preferably 0.05/hr or more and 4/hr or less, still more preferably 0.07/hr or more and 2.5/hr or less, yet still more preferably 0.07/hr or more and 2/hr or less, further preferably 0.07/hr or more and 1.5/hr or less, and further more preferably 0.07/hr or more and 1/hr or less.

<24> The method for producing an internal olefin according to any one of <1> to <23>, wherein an average double bond position of the internal olefin is 2 or more and 4.8 or less.

<25> The method for producing an internal olefin according to any one of <1> to <24>, wherein the average double bond position of the internal olefin is preferably 2 or more, more preferably 3 or more, still more preferably 3.5 or more, and yet still more preferably 4 or more, and preferably 4.8 or less, more preferably 4.6 or less, and still more preferably 4.5 or less.

<26> The method for producing an internal olefin according to any one of <1> to <25>, wherein the internal olefin having 16 carbon atoms and the internal olefin having 18 carbon atoms are preferably used in combination.

<27> A method for producing an internal olefin sulfonate,
the method comprising the step of sulfonating the internal olefin obtained by the method for producing an internal olefin according to any one of <1> to <26>.

<28> The method for producing an internal olefin sulfonate according to <27>, wherein the sulfonation step is the step of reacting the internal olefin with sulfur trioxide to obtain a sulfonated product.

<29> The method for producing an internal olefin sulfonate according to <28>, wherein sulfur trioxide is preferably caused to react as sulfur trioxide gas.

<30> The method for producing an internal olefin sulfonate according to <29>, wherein the sulfur trioxide gas is preferably diluted to 1 to 30 vol% with air and an inert gas such as nitrogen, and more preferably diluted to 1.5 to 10 vol%.

<31> The method for producing an internal olefin sulfonate according to any one of <28> to <30>, wherein the amount of sulfur trioxide used is preferably 0.8 mol or more, more preferably 0.9 mol or more, and still more preferably 0.95 mol or more, and is preferably 1.2 mol or less, more preferably 1.1 mol or less, and still more preferably 1.05 mol or less, based on 1 mol of the internal olefin.

<32> The method for producing an internal olefin sulfonate according to any one of <28> to <31>, wherein in the sulfonation reaction for causing the internal olefin to react with sulfur trioxide, a thin-film type sulfonation reactor provided with an external jacket is used.

<33> The method for producing an internal olefin sulfonate according to any one of <27> to <32>, wherein a treatment temperature in the sulfonation step is preferably 0°C or higher, and is preferably 50°C or lower.

<34> The method for producing an internal olefin sulfonate according to <32> or <33>, wherein it is to provide an external jacket in the sulfonation reactor and pass cooling water thereinto to cool the sulfonation reactor.

<35> The method for producing an internal olefin sulfonate according to <34>, wherein the temperature of the cooling water, which is passed into the external jacket of the sulfonation reactor is preferably 0 °C or higher, and preferably 30°C or lower, and more preferably 20°C or lower.

<36> The method for producing an internal olefin sulfonate according to <27> or <35>, wherein the sulfonation conversion ratio is preferably 95% or more, more preferably 97% or more, and still more preferably 98% or more, and is preferably 99.8% or less.

<37> The method for producing an internal olefin sulfonate according to <27> or <36>, comprising a step of neutralizing the obtained sulfonated product to obtain a neutralized product.

<38> The method for producing an internal olefin sulfonate according to <37>, wherein the alkali compound used for neutralization is preferably an inorganic alkali compound, more preferably an alkali metal hydroxide, still more preferably one selected from sodium hydroxide and potassium hydroxide, yet still more preferably sodium hydroxide, and yet still more preferably potassium hydroxide.

<39> The method for producing an internal olefin sulfonate according to <38>, wherein the alkali compound is used as an aqueous alkali solution.

<40> The method for producing an internal olefin sulfonate according to <39>, wherein the concentration of the aqueous alkali solution is preferably 1% by mass or more, more preferably 4.5% by mass or more, still more preferably 7% by mass or more, yet still more preferably 10% by mass or more, and further preferably 12% by mass or more, and preferably 30% by mass or less, more preferably 25% by mass or less, still more preferably 23% by mass or less, yet still more preferably 20% by mass or less, and further preferably 15% by mass or less.

<41> The method for producing an internal olefin sulfonate according to any one of <38> to <40>, wherein the amount of the alkali compound used is preferably 1 time by mole or more, and more preferably 1.03 times by mole or more, and preferably 2.5 times by mole or less, more preferably 2.0 times by mole or less, and still more preferably 1.5 times by mole or less, relative to the sulfonic acid group.

<42> The method for producing an internal olefin sulfonate according to any one of <37> to <41>, wherein in the neutralization step, a temperature when the sulfonate is mixed with the aqueous alkali solution and a temperature during the neutralization reaction are preferably 40°C or lower, more preferably 35°C or lower, still more preferably 30°C or lower, and yet still more preferably 25°C or lower, and preferably 0°C or higher, more preferably 10°C or higher, still more preferably 15°C or higher, and yet still more preferably 20°C or higher.

<43> The method for producing an internal olefin sulfonate according to any one of <37> to <42>, wherein in the neutralization step, the mixer is the agitating impeller type mixer.

<44> The method for producing an internal olefin sulfonate according to any one of <37> to <43>, wherein the neutralization step is preferably performed by a so-called continuous method in which the sulfonated product and the aqueous alkali solution are added while the reaction liquid is circulated using a loop type reactor, and at the same time, the reaction liquid is extracted.

<45> The method for producing an internal olefin sulfonate according to any one of <37> to <44>, wherein a neutralization time is preferably 5 minutes or more, more preferably 8 minutes or more, still more preferably 10 minutes or more, and yet still more preferably 12 minutes or more, and preferably 100 minutes or less, more preferably 50 minutes or less, still more preferably 40 minutes or less, yet still more preferably 30 minutes or less, further preferably 20 minutes or less, and further more preferably 15 minutes or less.

<46> The method for producing an internal olefin sulfonate according to <44> or <45>, wherein in the case of the continuous method, the average residence time is preferably 8 minutes or more, more preferably 10 minutes or more, and still more preferably 12 minutes or more, and preferably 100 minutes or less, more preferably 60 minutes or less, still more preferably 40 minutes or less, yet still more preferably 30 minutes or less, further preferably 20 minutes or less, and further more preferably 15 minutes or less.

<47> The method for producing an internal olefin sulfonate according to any one of <44> to <46>, wherein in the case of the continuous method, the circulation ratio of the reaction liquid is preferably 3 times or more, more preferably 6 times or more, and still more preferably 9 times or more, and preferably 30 times or less, more preferably 20 times or less, and still more preferably 15 times or less.

<48> The method for producing an internal olefin sulfonate according to any one of <37> to <47>, wherein in the neutralization step, a surfactant (coexisting product) is not used.

<49> The method for producing an internal olefin sulfonate according to any one of <37> to <47>, wherein in the case of using the surfactant (coexisting product) in the neutralization step, the surfactant is used in an amount of preferably 20% by mass or less, more preferably 10% by mass or less, still more preferably 2% by mass or less, and yet still more preferably 1% by mass or less in the mixed liquid.

<50> The method for producing an internal olefin sulfonate according to any one of <37> to <49>, comprising a hydrolysis step of hydrolyzing the obtained neutralized product.

<51> The method for producing an internal olefin sulfonate according to <50>, wherein a temperature during the hydrolysis is preferably 120°C or higher, more preferably 140°C or higher, and still more preferably 160°C or higher, and preferably 220°C or lower, and more preferably 180°C or lower.

<52> The method for producing an internal olefin sulfonate according to <50> or <51>, wherein the treatment time of the hydrolysis step is preferably 30 minutes or more, and more preferably 45 minutes or more, and is preferably 4 hours or less, more preferably 3.5 hours or less, still more preferably 3 hours or less, yet still more preferably 2 hours or less, and further preferably 90 minutes or less.

<53> The method for producing an internal olefin sulfonate according to any one of <50> to <52>, wherein the concentration of the aqueous internal olefin sulfonate solution obtained in the hydrolysis step is preferably 150 by mass or more, more preferably 30% by mass or more, still more preferably 35% by mass or more, yet still more preferably 40% by mass or more, further preferably 45% by mass or more, further more preferably 48% by mass or more, and further still more preferably 50% by mass or more, and is preferably 75% by mass or less, more preferably 70% by mass or less, still more preferably 65% by mass or less, and yet still more preferably 60% by mass or less.

<54> Use of the internal olefin sulfonate obtained by the method for producing an internal olefin sulfonate according to any one of <27> to <53> for body detergents, shampoos, clothing detergents, or tableware detergents.

<55> A method for stabilizing an internal olefin sulfonate at a low temperature,

the method comprising the steps of: sulfonating the internal olefin obtained by the method for producing an internal olefin according to any one of <1> to <26>; and neutralizing the sulfonated internal olefin to obtain the internal olefin sulfonate.

EXAMPLES

Hereinafter, the present invention is specifically described on the basis of Examples. In the following Examples and Comparative Examples, "%" means "% by mass" unless otherwise specified. Various measurement and evaluation methods are as follows.

<Method for measuring average particle diameter dp (sphere equivalent diameter) of solid acid catalyst particles>

50 solid acid catalyst particles were randomly extracted, and the minor axis diameters and major axis diameters of the particles were measured with a caliper. Using the arithmetic average value of the minor axis diameters and the arithmetic average value of the major axis diameters, the specific surface area of a cylinder was calculated with these values as the diameter of a bottom surface and a height. The diameter of a sphere having an equivalent specific surface area was defined as the average particle diameter dp (sphere equivalent diameter) of the solid acid catalyst particles.

<Method for measuring double bond distribution of internal olefin>

A double bond position in an internal olefin was measured by gas chromatography (hereinafter, abbreviated as GC). Specifically, the internal olefin was caused to react with dimethyl sulfide to prepare a dithionated derivative thereof, and individual components of the resultant derivative were separated from each other by GC. From the individual peak areas, double bond positions of the internal olefin were determined. An average double bond position was calculated by the following formula. An apparatus used for the measurement and the analysis conditions are as follows.

[Apparatus and measurement conditions]

GC apparatus: HP6890 (manufactured by Hewlett-Packard Company)
Column: Ultra-Alloy-1HT capillary column, 30 m $\times$ 250 um $\times$ 0.15 um (manufactured by Frontier Laboratories Ltd.)
Detector: hydrogen flame ion detector (FID)
Injection temperature: 300°C
Detector temperature: 350°C
He flow rate: 4.6 mL/min

[Calculation formula of average double bond position]

[Math 1]

Case where n is odd (average double bond position) =

$$\sum_{m=1}^{m=(n-1)/2} m \times Y/100 \qquad (1)$$

Case where n is even (average double bond position) =

$$\sum_{m=1}^{m=n/2} m \times Y/100 \qquad (2)$$

(In the formula, n represents the number of carbon atoms of an olefin, m represents a double bond position in the olefin, and Y represents the percentage of a m-olefin in an olefin having n carbon atoms.)

Example 1

(Production of 1-hexadecene)

Using a mono-tubular fixed bed reactor in which a reaction tube was installed in a vertical direction, γ-alumina as solid acid catalyst particles was packed up to the height of 0.4 m in the reaction tube. A temperature in the reaction tube was adjusted to 290°C, and 0.9 L (LHSV = 1/hr, WHSV = 1.1/hr) of 1-hexadecanol (product name: KALCOL 6098, manufactured by Kao Corporation) per hour and nitrogen were supplied at atmospheric pressure from the upper portion of the reactor so as to be 0.3 mol times with respect to 1-hexadecanol to perform an olefination reaction. A reaction tube outlet liquid was cooled to 70°C to recover crude 1-hexadecene. The obtained crude 1-hexadecene was transferred to a distillation flask and distilled at 150 to 160°C/9 mmHg to obtain 1-hexadecene having an olefin purity of 100%.

(Internal isomerization reaction)

Using a mono-tubular fixed bed reactor in which a reaction tube was installed in a vertical direction, γ-alumina as solid acid catalyst particles was packed up to the height of 0.2 m in the reaction tube. D/dp is 29. A temperature in the reaction tube was adjusted to 220°C, and the produced 1-hexadecene was supplied at 0.09 L per hour (LHSV = 0.2/hr, WHSV = 0.2/hr), and nitrogen was supplied at atmospheric pressure from the upper portion of the reactor so as to be 0.3 mol times with respect to 1-hexadecene to perform an internal isomerization reaction. A reaction tube outlet liquid was cooled to 70°C to recover an internal olefin having 16 carbon atoms. The average double bond position of the obtained internal olefin was 4.1.

(Method for producing internal olefin sulfonate having 16 carbon atoms (C16IOS))

The produced internal olefin having 16 carbon atoms was placed in a thin-film type sulfonation reactor having a jacket on the outside, and sulfur trioxide gas (1.1 vol%, balance air) was supplied under the condition that cooling water at 20°C was passed through the outer jacket of the reactor, to perform a sulfonation reaction. The molar ratio of $SO_3$/internal olefin during the sulfonation reaction was set to 1.005. The obtained sulfonated product was mixed with an aqueous alkali solution (potassium hydroxide: 17% by mass, balance water) prepared with potassium hydroxide (alkaline agent) in an amount 1.05 mole times the theoretical acid value. The mixture was neutralized at 30°C by a continuous method (using Milder mixer (product name: Milder MDN 303V, manufactured by MATSUBO Corporation), average residence time: 30 minutes, circulation ratio: 9 times). The neutralized product was hydrolyzed by heating in an autoclave at 170°C for 1 hour to obtain a liquid containing 55% by mass of potassium internal olefin sulfonate having 16 carbon atoms.

Examples 2 to 8 and Comparative Example 1

Internal olefins having 16 carbon atoms were produced in the same manner as in Example 1 except that conditions for internal isomerization reactions were changed to conditions described in Table 1. Using the obtained internal olefins having 16 carbon atoms, potassium internal olefin sulfonate-containing liquids having 16 carbon atoms were produced in the same manner as in Example 1.

Example 9

(Production of 1-octadecene)

Using a mono-tubular fixed bed reactor in which a reaction tube was installed in a vertical direction, γ-alumina as solid acid catalyst particles was packed up to the height of 0.4 m in the reaction tube. A temperature in the reaction tube was adjusted to 290°C, and 0.9 L (LHSV = 1/hr, WHSV = 1.1/hr) of 1-octadecanol (product name: KALCOL 8098, manufactured by Kao Corporation) per hour and nitrogen were supplied at atmospheric pressure from the upper portion of the reactor so as to be 0.3 mol times with respect to 1-octadecanol to perform an olefination reaction. A reaction tube outlet liquid was cooled to 70°C to recover crude 1-octadecene. The obtained crude 1-octadecene was transferred to a distillation flask and distilled at 175 to 180°C/9 mmHg to obtain 1-octadecene having an olefin purity of 100%.

(Internal isomerization reaction)

Using a mono-tubular fixed bed reactor in which a reaction tube was installed in a vertical direction, γ-alumina as solid acid catalyst particles was packed up to the height of 0.4 m in the reaction tube. D/dp is 29. A temperature in the reaction tube was adjusted to 245°C, and the produced 1-octadecene was supplied at 0.2 L per hour (LHSV = 1.9/hr, WHSV = 2.0/hr), and nitrogen was supplied at atmospheric pressure from the upper portion of the reactor so as to be 0.3 mol times with respect to 1-octadecene to perform an internal isomerization reaction.
A reaction tube outlet liquid was cooled to 70°C to recover an internal olefin having 18 carbon atoms. The average double bond position of the obtained internal olefin was 4.0.

(Method for producing internal olefin sulfonate having 18 carbon atoms (C18IOS))

The produced internal olefin having 18 carbon atoms was placed in a thin-film type sulfonation reactor having a jacket on the outside, and sulfur trioxide gas (1.1 vol%, balance air) was supplied under the condition that cooling water at 20°C was passed through the outer jacket of the reactor, to perform a sulfonation reaction. The molar ratio of $SO_3$/internal olefin during the sulfonation reaction was set to 1.005. The obtained sulfonated product was mixed with an aqueous alkali solution (potassium hydroxide: 17% by mass, balance water) prepared with potassium hydroxide (alkaline agent) in an amount 1.05 mole times the theoretical acid value. The mixture was neutralized at 30°C by a continuous method (using Milder mixer (product name: Milder MDN 303V, manufactured by MATSUBO Corporation), average residence time: 30 minutes, circulation ratio: 9 times). The neutralized product was hydrolyzed by heating in an autoclave at 170°C for 1 hour to obtain a liquid containing 55% by mass of potassium internal olefin sulfonate having 18 carbon atoms.

Examples 10 to 12 and Comparative Examples 2 and 3

Internal olefins having 18 carbon atoms were produced in the same manner as in Example 9 except that conditions for internal isomerization reactions were changed to conditions described in Table 2. Using the obtained internal olefins having 18 carbon atoms, potassium internal olefin sulfonate-containing liquids having 18 carbon atoms were produced in the same manner as in Example 9.
The solid acid catalyst particles (γ-alumina) used in the above Examples had a particle diameter dp of about 1 to 5 mm. Each of the reaction tubes in which the solid acid catalyst particles were packed had a single diameter, and an inner diameter D of about 50 to 2000 mm.

<Evaluation of low-temperature stability>

100 g of each of the produced potassium internal olefin sulfonate-containing liquids was placed in a wide-mouth standard bin PS-13K, and sealed. The sealed liquid was left to stand in a thermostatic chamber at -5°C for 1 week. The appearance of potassium internal olefin sulfonate after one week was determined according to the following criteria.

    A: No change was observed in appearance at all.
    B: A very small amount of precipitate was observed.
    C: A small amount of precipitate was observed.
    D: A lot of precipitates were deposited at the bottom of the bin.

[Table 1]

| | | Example 1 | Example 2 | Example 3 | Example 4 | Example 5 | Example 6 | Example 7 | Example 8 | Comparative Example 1 |
|---|---|---|---|---|---|---|---|---|---|---|
| Production conditions of internal olefin | Number of carbon atoms of raw material alcohol | 16 | 16 | 16 | 16 | 16 | 16 | 16 | 16 | 16 |
| | D/dp | 29 | 29 | 412 | 792 | 792 | 792 | 792 | 29 | 6.9 |
| | Catalyst packing height [mm] | 200 | 200 | 200 | 6000 | 6000 | 6000 | 6000 | 200 | 100 |
| | WHSV during internal isomerization reaction | 0.2 | 1 | 1 | 0.17 | 0.17 | 0.17 | 0.09 | 3 | 11 |
| | LHSV during internal isomerization reaction | 0.2 | 1 | 1 | 0.16 | 0.16 | 0.16 | 0.08 | 3 | 10 |
| | Reaction temperature during internal isomerization reaction (°C) | 220 | 250 | 228 | 215 | 180 | 203 | 202 | 278 | 280 |
| Measurement and evaluation results | Average double bond position of internal olefin | 4.1 | 4.1 | 4.2 | 4.1 | 4.1 | 4.1 | 4.2 | 4.1 | 4.1 |
| | Low-temperature stability | B | B | A | A | A | A | A | C | D |

EP 4 378 918 A1

[Table 2]

| | | Example 9 | Example 10 | Example 11 | Example 12 | Comparative Example 2 | Comparative Example 3 |
|---|---|---|---|---|---|---|---|
| Production conditions of internal olefin | Number of carbon atoms of raw material alcohol | 18 | 18 | 18 | 18 | 18 | 18 |
| | D/dp | 29 | 412 | 792 | 792 | 22 | 11 |
| | Catalyst packing height [mm] | 400 | 2000 | 6000 | 6000 | 400 | 200 |
| | WHSV during internal isomerization reaction | 1.4 | 1 | 0.17 | 0.12 | 0.9 | 2 |
| | LHSV during internal isomerization reaction | 1.3 | 1 | 0.15 | 0.11 | 0.9 | 2 |
| | Reaction temperature during internal isomerization reaction [°C] | 245 | 216 | 170 | 170 | 245 | 260 |
| Measurement and evaluation results | Average double bond position of internal olefin | 4.0 | 4.1 | 4.0 | 4.0 | 4.0 | 3.9 |
| | Low-temperature stability | B | A | A | A | D | D |

INDUSTRIAL APPLICABILITY

The internal olefin obtained by the production method of the present invention is useful as a petroleum drilling oil base oil, a detergent raw material, a paper sizing agent raw material, a base oil or a raw material of a lubricating oil, and a chemical product raw material and the like. The internal olefin sulfonate obtained by the production method of the present invention is useful as a base for various detergents.

**Claims**

1. A method for producing an internal olefin,
   the method comprising the step of internally isomerizing an $\alpha$-olefin having 8 or more and 24 or less carbon atoms using a fixed bed reactor in which solid acid catalyst particles are packed in a reaction tube, wherein a ratio (D/dp) of an inner diameter D of the reaction tube to an average particle diameter dp of the solid acid catalyst particles is 25 or more.

2. The method for producing an internal olefin according to claim 1, wherein a reaction temperature when the $\alpha$-olefin is internally isomerized is 300°C or lower.

3. The method for producing an internal olefin according to claim 1 or 2, wherein in the internal isomerization step, WHSV ($\alpha$-olefin supply mass per hour per solid acid catalyst unit mass) is 0.03/hr or more and 7/hr or less.

4. The method for producing an internal olefin according to any one of claims 1 to 3, wherein in the internal isomerization step, LHSV (liquid hourly space velocity) is 0.03/hr or more and 5/hr or less.

5. The method for producing an internal olefin according to any one of claims 1 to 4, wherein an average double bond position of the internal olefin is 2 or more and 4.8 or less.

6. A method for producing an internal olefin sulfonate,
   the method comprising the step of sulfonating the internal olefin obtained by the method for producing an internal olefin according to any one of claims 1 to 5.

7. A method for stabilizing an internal olefin sulfonate at a low temperature,
   the method comprising the steps of: sulfonating the internal olefin obtained by the method for producing an internal olefin according to any one of claims 1 to 5; and neutralizing the sulfonated internal olefin to obtain the internal olefin sulfonate.

## INTERNATIONAL SEARCH REPORT

| International application No. |
| --- |
| **PCT/JP2022/028887** |

**A. CLASSIFICATION OF SUBJECT MATTER**

*C07C 5/25*(2006.01)i; *C07C 11/02*(2006.01)i; *C07C 303/06*(2006.01)i; *C07C 303/32*(2006.01)i; *C07C 309/20*(2006.01)i; *C07B 61/00*(2006.01)i; *B01J 21/04*(2006.01)i

FI: C07C5/25; C07C11/02; C07C309/20; C07C303/32; B01J21/04 Z; C07C303/06; C07B61/00 300

According to International Patent Classification (IPC) or to both national classification and IPC

**B. FIELDS SEARCHED**

Minimum documentation searched (classification system followed by classification symbols)

C07C5/25; C07C11/02; C07C303/06; C07C303/32; C07C309/20; C07B61/00; B01J21/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2014-144945 A (KAO CORP) 14 August 2014 (2014-08-14) claims, paragraphs [0011], [0019], examples | 1-5 |
| Y | | 1-7 |
| X | JP 2014-156462 A (KAO CORP) 28 August 2014 (2014-08-28) claims, examples | 1-7 |
| Y | | 1-7 |
| X | JP 2012-24766 A (CHEVRON ORONITE CO LLC) 09 February 2012 (2012-02-09) in particular, examples | 1-5 |
| Y | | 1-7 |
| Y | JP 2004-519419 A (CHEVRON U.S.A. INC.) 02 July 2004 (2004-07-02) paragraphs [0010], [0040], [0044] | 1-7 |
| Y | JP 2013-241386 A (KAO CORP) 05 December 2013 (2013-12-05) claims, paragraph [0013], examples | 1-7 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **22 September 2022** | **04 October 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2022/028887** |

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2011-500628 A (BASF SE) 06 January 2011 (2011-01-06)<br>in particular, examples | 1-7 |
| Y | JP 2020-97544 A (JX NIPPON OIL & ENERGY CORP) 25 June 2020 (2020-06-25)<br>in particular, examples | 1-7 |
| Y | JP 2014-213290 A (TOKYO INST TECH) 17 November 2014 (2014-11-17)<br>in particular, examples | 1-7 |
| Y | JP 2017-87107 A (JX NIPPON OIL & ENERGY CORPORATION) 25 May 2017<br>(2017-05-25)<br>in particular, examples | 1-7 |
| Y | JP 2008-222609 A (IDEMITSU KOSAN CO LTD) 25 September 2008 (2008-09-25)<br>in particular, examples | 1-7 |
| Y | JP 2006-193442 A (IDEMITSU KOSAN CO LTD) 27 July 2006 (2006-07-27)<br>in particular, examples | 1-7 |
| Y | US 5321193 A (CHINESE PETROLEUM CORPORATION) 14 June 1994 (1994-06-14)<br>column 3, lines 54-66 | 1-7 |

Form PCT/ISA/210 (second sheet) (January 2015)

**INTERNATIONAL SEARCH REPORT**
Information on patent family members

International application No.

**PCT/JP2022/028887**

| Patent document cited in search report | | | Publication date (day/month/year) | Patent family member(s) | | | Publication date (day/month/year) |
|---|---|---|---|---|---|---|---|
| JP | 2014-144945 | A | 14 August 2014 | (Family: none) | | | |
| JP | 2014-156462 | A | 28 August 2014 | WO | 2014/112522 | A1 | |
| | | | | claims, examples | | | |
| | | | | CN | 104918902 | A | |
| JP | 2012-24766 | A | 09 February 2012 | US | 2005/0203322 | A1 | |
| | | | | examples | | | |
| | | | | EP | 2295392 | A2 | |
| | | | | CA | 2497958 | A | |
| | | | | SG | 115751 | A | |
| JP | 2004-519419 | A | 02 July 2004 | US | 2002/0188031 | A1 | |
| | | | | paragraphs [0010], [0050], [0057] | | | |
| | | | | GB | 2386123 | A | |
| | | | | WO | 2002/008163 | A1 | |
| | | | | AU | 5449501 | A | |
| | | | | NL | 1018618 | A | |
| | | | | BR | 112705 | A | |
| JP | 2013-241386 | A | 05 December 2013 | (Family: none) | | | |
| JP | 2011-500628 | A | 06 January 2011 | US | 2010/0286458 | A1 | |
| | | | | examples | | | |
| | | | | WO | 2009/050194 | A1 | |
| | | | | EP | 2212267 | A1 | |
| | | | | KR | 10-2010-0075997 | A | |
| | | | | CN | 101827804 | A | |
| JP | 2020-97544 | A | 25 June 2020 | US | 2022/0064085 | A1 | |
| | | | | examples | | | |
| | | | | WO | 2020/130056 | A1 | |
| JP | 2014-213290 | A | 17 November 2014 | (Family: none) | | | |
| JP | 2017-87107 | A | 25 May 2017 | US | 2019/0134613 | A1 | |
| | | | | examples | | | |
| | | | | WO | 2017/077863 | A1 | |
| JP | 2008-222609 | A | 25 September 2008 | (Family: none) | | | |
| JP | 2006-193442 | A | 27 July 2006 | US | 2008/0167510 | A1 | |
| | | | | examples | | | |
| | | | | WO | 2006/075526 | A1 | |
| | | | | EP | 1842840 | A1 | |
| | | | | CA | 2593310 | A | |
| US | 5321193 | A | 14 June 1994 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014156462 A